# EUROPEAN PATENT SPECIFICATION

(11) **EP 1 888 001 B1**
(45) Date of publication and mention of the grant of the patent: **06.08.2014**
(21) Application number: 06756211.6
(22) Date of filing: 11.06.2006
(51) Int. Cl.: A61K 9/70, A61F 13/02

(54) **PATCH FOR TRANSDERMAL DRUG DELIVERY**
PATCH ZUR TRANSDERMALEN WIRKSTOFFFREISETZUNG
TIMBRE TRANSDERMIQUE POUR LA DELIVRANCE DE MEDICAMENTS

(30) Priority: 10.06.2005 US 689763 P
(43) Date of publication of application: 20.02.2008
(73) Proprietor: Syneron Medical Ltd., 20692 Yoqneam Illit (IL)
(72) Inventor: LEVIN, Galit, Nordiya (IL); STERN, Meir, Rehovot (IL); SACKS, Hagit, Modi'in (IL); ARBEL, Giora, 40600 Tel Mond (IL); BAR EL, Yossi, Beit Arie 71947 (IL)
(74) Representative: Jennings, Tara Romaine
(86) International application number: PCT/IL2006/000679
(87) International publication number: WO 2006/131931

(56) References cited:
- WO-A1-94/14062
- WO-A1-02/090210
- US-A- 4 837 027
- US-A- 5 698 217
- US-A1- 2001 051 180
- US-B1- 6 169 224

## Description

### FIELD OF THE INVENTION

The present invention relates generally to techniques for drug delivery, and specifically to methods and apparatus for transdermal drug delivery.

### BACKGROUND OF THE INVENTION

Adhesive transdermal drug patches deliver a drug across the skin directly into the systemic blood circulation. Typically, the drug is dispersed in the adhesive that attaches the patch to the skin.

PCT Publication WO 03/039620 to Sohn, describes apparatus for facilitating delivery of a substance through skin of a subject The apparatus includes a handle and a cartridge, removably coupled to the handle. The cartridge includes one or more electrodes and a patch comprising the substance, the electrodes adapted to be applied to a region of the skin, and the patch adapted to be applied to at least a portion of the region of the skin by removal of the electrodes therefrom. Also described is a medicated patch which comprises a lower backing, a pad, and an upper protective cover covering pad. The pad has two sections: (a) an electrical treatment side, having a shaped portion such as a frame that surrounds an open portion passing therethrough, and (b) a substance-treatment side, having a medicated region. The lower backing covers an adhesive region surrounding the open portion on the underside of the electrical treatment side (which underside comes in contact with the skin). The upper cover protects the upper portion of pad, including the substance-containing region, from contamination. The upper cover comprises a tab, connected to an edge near the open portion.

US Patent 5,603,693 to Frenkel et al., describes a device having three separable modules, for the transdermic administration of drugs by electrophoresis or iontophoresis, which comprises a first active module provided with at least one system of electrodes and one drug reservoir, a second power module provided with a power supply, and a third electronic module having an electronic circuit, control organs, and a display screen. The power module is situated between the two other modules and comprises, in addition to the power supply formed by one or more batteries, mechanical assembly means and electrical connection or interconnection means with the two other modules means for attaching the device to the body of a patient.

US Patent 5,908,401 to Henley, describes a portable iontophoresis apparatus for facilitating delivery of medication across the cutaneous membrane into adjacent underlying tissues and blood vessels. The apparatus employs a modular, detachable non-reusable medicament-containing applicator electrode which is adapted to attach to a base assembly. The apparatus is designed to be hand-held and includes a circumferential tactile electrode band on the base assembly which provides electrical connection between the skin of the user's hand and one pole of a bipolar power source housed within the base assembly. The opposing pole of the power source is connected to the applicator electrode. The user's body completes the electrical circuit between the applicator and tactile electrodes.

US Patent 5,919,156 to Stropkay et al., describes an iontophoretic drug delivery system including a plurality of patches, at least one reusable controller, and a unit for storing and dispensing the patches. The patches may be secured in a compartment formed in the unit and the controller may be stored in another compartment formed in the unit. In this way, the reusable controller and a new patch can be removed from the unit and fastened to one another for activation and attachment to the skin of a patient.

US Patent 3,163,166 to Brant et al., describes a method for the treatment by iontophoresis of a selected area of soft tissue surface, including passing an electric current between a selected area of soft tissue surface and an external electrode. The current is passed through electrolyte interposed and maintained between the external electrode and the area of soft tissue surface while keeping the electrode in motion over the area.

US 2001/051180 discloses a transdermal drug delivery device with a non-occlusive backing or release liner in combination with a degradation protectant such as a desiccant or oxygen scavenger within a sealed pouch containing the device.

US 5,698,217 discloses a transdermal drug delivery device involving a carrier containing a dissolved drug. The device also involves a desiccant package that is inert to the carrier, permeable to water vapour, and defines a desiccant compartment containing a desiccant. The device also involves a water vapour impermeable product package that contains the carrier and the desiccant package.

WO 02/090210 discloses a device and method for stabilizing a drug, particularly a chiral drug or the active enantiomer(s) thereof, in a carrier composition of a transdermal delivery system prior to the system's use, by providing a product packaging system to prevent or control degradation reactions that can result from certain packaging materials and moisture contamination, while at the same time providing a child-resistant wrapping for the transdermal system.

WO 94/14062 discloses a dermal patch to be worn on the skin for increasing the concentration of an analyte expressed through the skin in perspiration to a conveniently measurable level. Included are patches and methods of using such patches to determine the quantity of an analyte in a given volume of perspiration, to determine a subject's sensitivity to an allergen, to allow an analyte to be detected with conventional detection systems, and to dissolve the structural components of a patch to facilitate analyte detection.

### SUMMARY OF THE INVENTION

According to the present invention there is provided an apparatus as specified in claim 1 and a method as specified in claim 13.

In embodiments of the present invention, a transdermal drug delivery patch product comprises a patch and protective packaging for storing and protecting the patch prior to use. The protective packaging typically comprises a package, in which the patch is stored. During manufacture, the drug is applied to the patch in liquid form, such as by using a printing-like process or another technique known in the art. The drug is typically allowed to partially dry for a short time before being placed in the package. The protective packaging is configured to allow the drug to continue drying on the patch after the patch has been inserted into the package. Such post-packaging drying typically reduces manufacturing time, complexity, and/or cost. The drug generally has greater stability and shelf life when in a substantially dry form than in a liquid or only partially-dry form.

In some embodiments of the present invention, the patch comprises a backing liner having an adhesive area and a drug delivery area. The protective packaging comprises (in addition to the package) a removable protective covering applied to the patch. The protective covering is configured to substantially not come in contact with the drug delivery area of the patch, and is shaped so as to define one or more holes therethrough. The package stores the patch together with the protective covering prior to use. The package typically comprises a moisture-absorbing desiccant, such as a silica gel desiccant. The desiccant is in fluid communication, via the holes, with the drug delivery area of the patch. The desiccant thus absorbs moisture from the drug while it is in its partially-dry form, thereby completing the drying of the drug to a level of dryness suitable for long-term storage.

It is noted that different drugs typically have different moisture content levels in the partially-dry form, and also have different moisture content levels that are suitable for long-term storage. Nevertheless, by way of illustration and not limitation, some embodiments of the invention include reducing the moisture content to a partially-dry level of between about 6% and 10% or between about 10% and 15% by weight before placing the patch into the package. Alternatively or additionally, embodiments of the invention include reducing the moisture content from the partially-dry level to between about 2% and 3% or between about 3% and 4% or between about 4% and 5%, after the patch has been placed in the package.

Typically, the protective covering is held to the patch by the adhesive area, which is also used to attach the patch to the skin.

There is therefore provided, in accordance with an embodiment of the present invention, apparatus including a transdermal drug delivery patch product, which includes:
a patch, which includes a drug; and
protective packaging, adapted to store the patch, and to allow the drug to dry while the patch is stored in the protective packaging.

For some applications, the protective packaging is configured to dry the drug to a moisture content of 2-3% or 3-5% by weight while the patch is stored in the protective packaging.

In an embodiment, the patch is shaped so as to defme: a first portion shaped so as to define a frame that surrounds a window, and a second portion, adjacent to the first portion, that defines a drug delivery area including the drug, and the patch is configured such that when the second portion is folded onto the first portion, the drug delivery area is placed through the window.

In an embodiment, the protective packaging includes a package, adapted to store the patch, and the package includes a desiccant in fluid communication with the drug when the patch is stored in the package.

In an embodiment, the protective packaging includes a removable protective covering applied to the patch, configured such that during storage, when the protective covering is applied to the patch, at least a portion of the protective covering is spaced away from the drug, and the protective covering is shaped so as to define one or more holes therethrough. For some applications, the protective packaging includes a package, adapted to store the patch together with the protective covering, and including a desiccant in fluid communication with the drug via the holes when the patch is stored in the package. For some applications, the protective covering includes exactly one element. For some applications, the element is shaped so as to define a peripheral area, and a raised central area shaped so as not to come in contact with the drug.

In an embodiment, the protective packaging includes a removable protective covering applied to the patch, configured such that during storage, when the protective covering is applied to the patch, at least a portion of the protective covering is spaced away from the drug, and the protective covering includes a desiccant. For some applications, the desiccant is disposed on a face of the protective covering that faces the patch. Alternatively, for some applications, the protective covering is shaped so as to define one or more holes therethrough, and the desiccant is disposed on a face of the protective covering that faces away from the patch, such that the desiccant is in fluid communication with the drug via the holes when the patch is stored in the protective packaging.

For some applications, the protective covering is shaped so as to define between 1 and 10 holes therethrough, between 10 and 100 holes therethrough, or greater than 100 holes therethrough.

For some applications, the protective covering includes at least one item selected from the list consisting of: a cloth, a plastic, a screen, a mesh, a porous material, and a moisture-permeable film, and the one or more holes are holes in the selected item.

There is further provided, in accordance with an embodiment of the present invention, a method for manufacturing a transdermal drug delivery patch product, the method including:
applying a drug to a patch; and
while at least a portion of the drug has greater than 6% moisture content by weight, storing the patch in protective packaging adapted to dry the drug while the patch is stored in the protective packaging.

In an embodiment, storing the patch in the protective packaging includes placing a desiccant in the protective packaging.

For some applications, storing the patch includes storing the patch in the protective packaging, the protective packaging being adapted to dry the drug to a moisture content of 2-6% or 3-5% by weight while the patch is stored in the protective packaging.

In an embodiment, the protective packaging includes a package which includes a desiccant, and storing the patch includes storing the patch in the package such that the desiccant is in fluid communication with the drug. For some applications, the protective packaging includes a removable protective covering shaped so as to define one or more holes therethrough, and storing the patch includes applying the protective covering to the patch such that at least a portion of the protective covering is spaced away from the drug. Alternatively, for some applications, the protective packaging includes a package which includes a desiccant, and storing the patch includes storing the patch in the package together with the protective covering such that the desiccant is in fluid communication with the drug via the holes.

For some applications, the protective packaging includes a removable protective covering including a desiccant, and storing the patch includes applying the protective covering to the patch such that at least a portion of the protective covering is spaced away from the drug. For some applications, the desiccant is disposed on a face of the protective covering that faces the patch. For some applications, the protective covering is shaped so as to define one or more holes therethrough, the desiccant is disposed on a face of the protective covering that faces away from the patch, and storing the patch includes applying the protective covering to the patch such that the desiccant is in fluid communication with the drug via the holes.

There is still further provided, in accordance with an embodiment of the present invention, a method for transdermally administering a drug to a subject, including:
applying a drug to a patch;
while at least a portion of the drug has greater than 6% moisture content by weight, storing the patch in protective packaging;
allowing the drug to dry while the patch is stored in the package; and
after the drug dries, applying the patch to skin of the subject, such that moisture from the skin dissolves the drug.

In an embodiment, the patch is shaped so as define a first portion and a second portion adjacent to the first portion, and applying the patch to the skin includes:
adhering a frame of the first portion to the skin such that a portion of the skin is exposed through a window defined by the frame; and
folding the second portion onto the first portion, such that a drug delivery area of the second portion that includes the drug is applied through the window to the portion of the skin.

In an embodiment, storing the patch in the protective packaging includes placing a desiccant in the protective packaging.

In an embodiment, the protective packaging includes a removable protective covering shaped so as to define one or more holes therethrough, storing the patch includes applying the protective covering to the patch such that at least a portion of the protective covering is spaced away from the drug, and applying the patch includes removing the protective covering from the patch.

In an embodiment, the protective packaging includes a package which includes a desiccant, and storing the patch includes storing the patch in the package together with the protective covering such that the desiccant is in fluid communication with the drug via the holes.

The present invention will be more fully understood from the following detailed description of embodiments thereof, taken together with the drawings, in which:

### BRIEF DESCRIPTION OF THE DRAWINGS

Figs. 1A-D are schematic illustrations of a transdermal drug delivery product, in accordance with an embodiment of the present invention;
Figs. 2A-C are schematic illustrations of another transdermal drug delivery product, in accordance with an embodiment of the present invention;
Figs. 3A-D are schematic illustrations of application of a patch of the product of Figs. 1A-D or Figs. 2A-C to skin of a subject, in accordance with an embodiment of the present invention;
Figs. 4, 5, and 6A-B are schematic illustrations of yet another transdermal drug delivery product, in accordance with an embodiment of the present invention; and
Figs. 7A-E are schematic illustrations of application of a patch of the product of Figs. 4, 5, and 6A-B to skin of a subject, in accordance with an embodiment of the present invention.

### DETAILED DESCRIPTION OF EMBODIMENTS

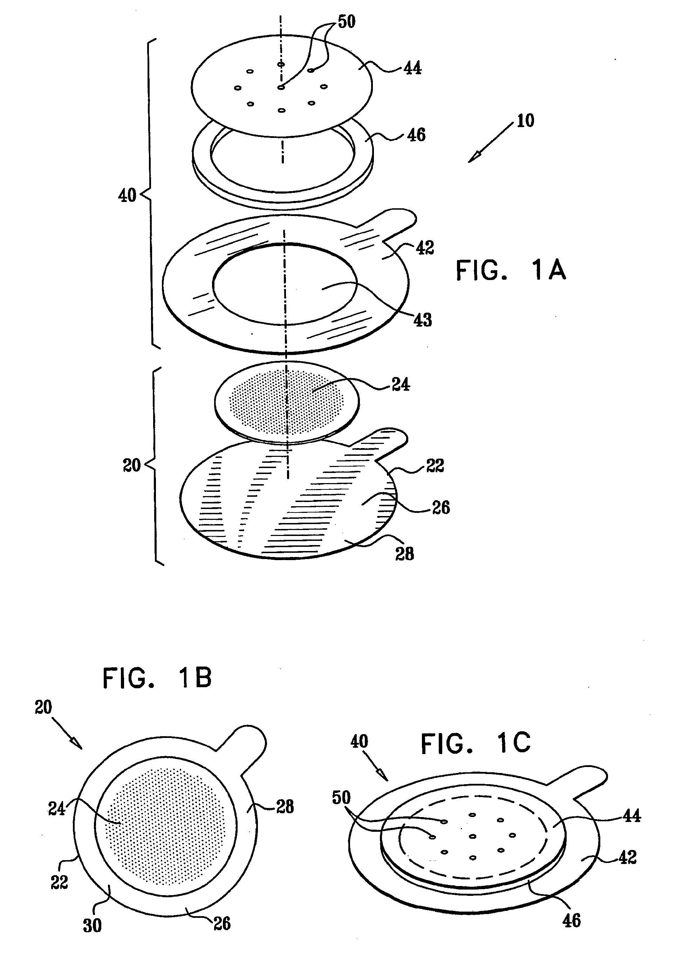

Figs. 1A-D are schematic illustrations of a transdermal drug delivery product 10, in accordance with an embodiment of the present invention. Fig. 1A shows a portion of the elements of product 10 prior to assembly. Product 10 comprises a patch 20, which typically comprises a backing liner 22 and a drug delivery area 24. A surface 26 of backing liner 22 typically comprises an adhesive 28. As shown in Fig. 1B, a portion of adhesive 28 typically secures drug delivery area 24 to surface 26, while the remainder of adhesive 28 remains exposed and defines an adhesive area 30, typically around the periphery of backing liner 22. Adhesive area 30 typically serves both to secure patch 20 to a removable protective covering 40, described hereinbelow, and to secure the patch to skin of a subject after the protective covering has been removed. The scope of the invention includes functionally equivalent alternative configurations of patch 20, such as forming drug delivery area 24 as an integrated component of backing liner 22, e.g., by applying a drug directly to a portion of backing liner 22.

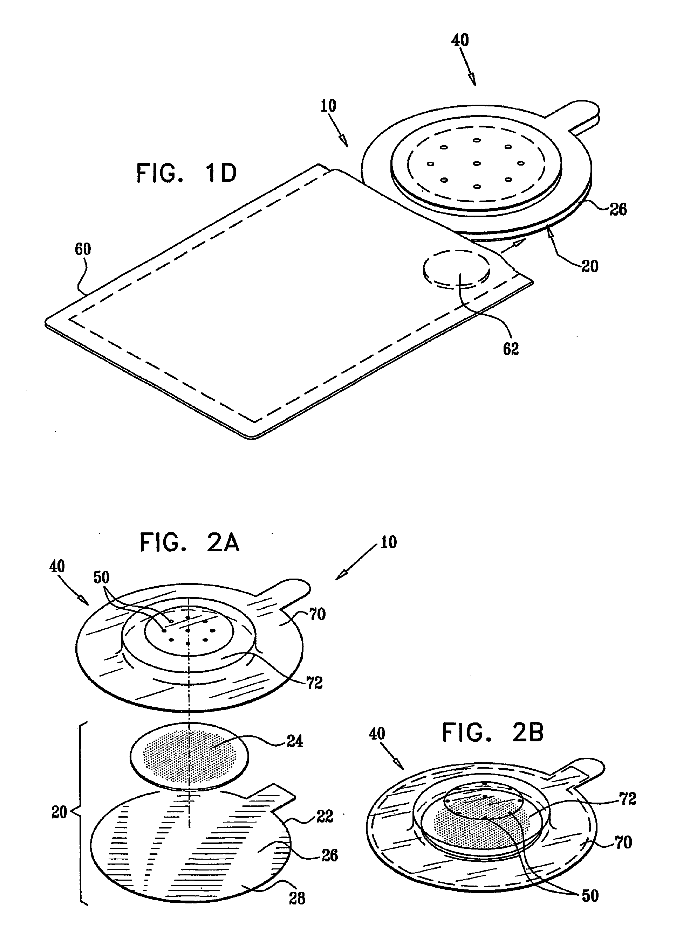

Reference is made to Figs. 1A and 1C, which show the elements of protective covering 40 unassembled and assembled, respectively. Protective covering 40 is configured to substantially not come in contact with drug delivery area 24 when the protective covering is applied to patch 20. For some applications, protective covering 40 comprises: (a) a lower layer 42, which is brought into contact with adhesive area 30 of backing liner 22, and which typically defines a central opening 43 therethrough, (b) an upper layer 44, which provides protection for drug delivery area 24 from mechanical contact with the environment, and (c) a spacing layer 46, which prevents upper layer 44 from coming in contact with drug delivery area 24 or minimizes the likelihood of such contact under normal handling conditions. For example, a plurality of transdermal drug delivery products 10 may be stored in a box intended for retail sale, such that the box prevents undue forces from being applied to each package 60, as described hereinbelow with reference to Fig. 1D (and thereby minimizes the likelihood of contact between upper layer 44 and drug delivery area 24 in each package). The scope of the invention includes functionally equivalent alternative configurations of protective covering 40. For example, lower layer 42 and upper layer 44 may comprise a single protective layer, and spacing layer 46 may be coupled to a lower surface of the single protective layer, so as to prevent contact between a central portion of the single protective layer and drug delivery area 24 (configuration not shown).

Upper layer 44 of protective covering 40 is typically configured to allow gas passage therethrough. Typically, upper layer 44 is shaped so as to define one or more holes 50 therethrough for this purpose. The gas within package 60 is typically inert, and may comprise, as appropriate, nitrogen and/or argon. Alternatively, the gas comprises air or another combination of gases.

Reference is made to Fig. 1D, which shows the complete product 10 after assembly, including patch 20 and protective covering 40, as described hereinabove, and a package 60 for storing patch 20 together with protective covering 40. Package 60 typically comprises a moisture-absorbing desiccant 62, such as a silica gel desiccant. For example, desiccant 62 may comprise a conventional desiccant bag or other desiccant container typically used in drug packaging. For some applications, about 0.25 g to about 3 g of desiccant 62 is provided (e.g., about 0.5 g to about 2 g). Desiccant 62 may be fixed to or removable from package 60, as appropriate. Alternatively or additionally, desiccant 62 is fixed to or integrated with protective covering 40. For some applications, protective covering 40 has no holes, and the desiccant is on the face of protective covering 40 that faces the drug. For other applications, protective covering 40 has holes, and the desiccant is on the face of protective covering 40 that is away from the drug, whereby the holes provide fluid communication between the desiccant and the drug.

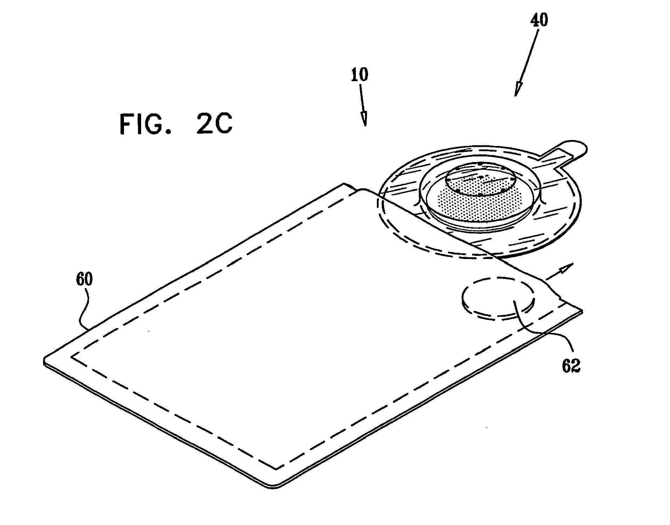

Reference is made to Figs. 2A-C, which are schematic illustrations of another embodiment of transdermal drug delivery product 10. Except as described below, this embodiment is the same as the embodiment described hereinabove with reference to Figs.1A-D. In this embodiment, protective covering 40 comprises a single element, which is shaped so as to define: (a) a peripheral area 70, which is brought into contact with adhesive area 30 of backing liner 22, and (b) a raised central area 72, which provides protection for drug delivery area 24 from mechanical contact with the environment, and is shaped so as not to come into contact with drug delivery area 24, or so as to minimize the likelihood of such contact under normal handling conditions. Raised central area 72 of protective covering 40 is typically configured to allow gas passage therethrough. Typically, raised central area 72 is shaped so as to define one or more holes 50 therethrough for this purpose.

For some applications, protective covering 40 comprises a thermoformable film (e.g., PVC, PETG, or HDPE), which is coated on one side, such as siliconized, and then formed to the desired blister shape using a vacuum-forming process. The film typically has a thickness of between about 150 and about 300 microns. For applications in which adhesive area 30 comprises an acrylic pressure-sensitive adhesive (PSA), the silicone coating provides a release peeling force with the adhesive layer of between about 10 and 30 g per two-inch strip when pulled at 180 degrees, and thus enables easy release while providing good protection to adhesive area 30 and drug delivery area 24. Alternatively, protective covering 40 is manufactured by forming an uncoated film to the desired blister shape, and then laminating or coating the blister rim (the lower side) with a silicone layer.

Reference is made to Figs. 1A-D and 2A-C. In an embodiment of the present invention, during manufacture of product 10, a drug in a liquid form is applied to drug delivery area 24, such as by using a process like that which is used in ink-jet printing. For some applications, the drug is in a suspension and/or solution when it is applied to area 24. For some applications, the drug is applied as a plurality of small droplets on drug delivery area 24 by a syringe that moves over the surface of area 24 or stays stationary while area 24 moves thereunder. Alternatively or additionally, the drug is applied to area 24 using a technique that is known in the art, such as spraying, rolling, or coating the drug onto area 24. For greater stability and shelf life of the drug, it is desirable that the drug be sufficiently dry during storage of product 10. However, many drugs dry slowly after application to the drug delivery area (for example, some drugs do not dry sufficiently for long-term stability of the drug until up to about 1-4 weeks after application to the drug delivery area). Waiting for the drug to dry sufficiently for long-term stability generally increases manufacturing cost and complexity. Use of protective covering 40 and package 60, as described hereinabove, allows patch 20 to be packaged immediately upon application of the drug, without waiting for the drug to dry, or within about 30-120 minutes (e.g., 30-60 minutes) following application of the drug, while the drug is only partially dry. While packaged, the drug is in fluid (i.e., gaseous) communication, via holes 50, with desiccant 62, which absorbs moisture from the drug, thereby drying the drug to a level sufficient for long-term storage (typically within about 1-4 weeks of packaging). At the same time, upper layer 44 of protective covering 40 protects the drug from mechanical contact with the environment, including the inside of package 60.

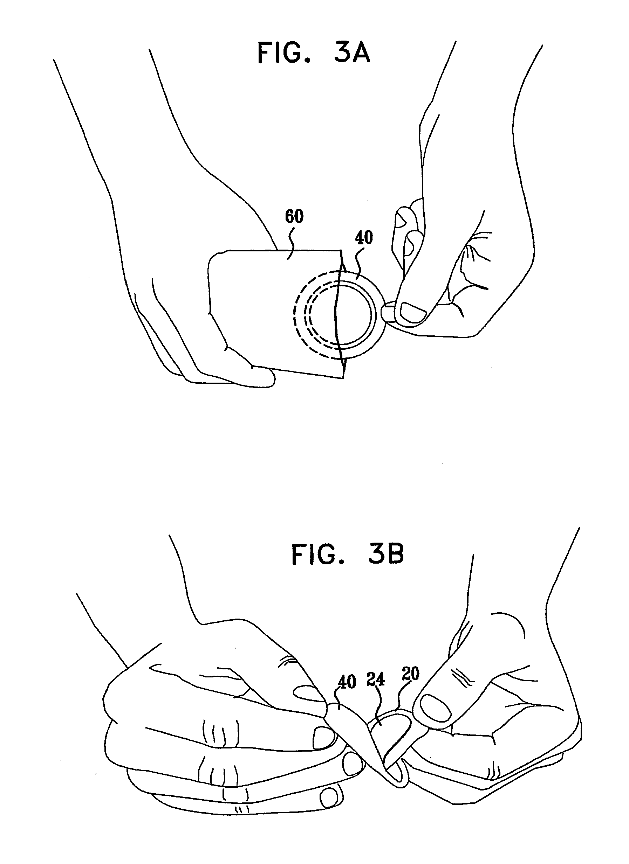

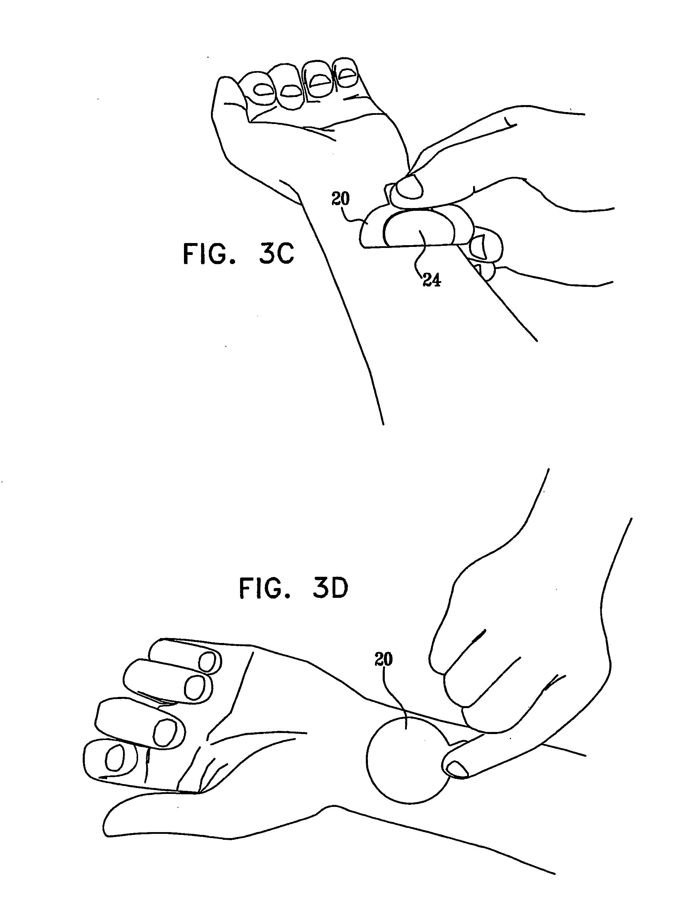

Reference is made to Figs. 3A-D, which are schematic illustrations of application of patch 20 to skin of a subject, in accordance with an embodiment of the present invention. Fig. 3A shows the subject removing patch 20, together with protective covering 40, from package 60. The subject then peels patch 20 apart from protective covering 40, as shown in Fig. 3B. The subject applies the patch to skin, as shown in Figs. 3C and 3D. In an embodiment, drug delivery area 24 protrudes about 1-3 or about 3-5 mm from patch 20 (Figs. 1A, 3B, and 3C), such that the application of patch 20 to the skin enhances the contact pressure between drug delivery area 24 and the skin.

In an embodiment, transdermal drug delivery product 10 is adapted to be used without any particular skin-preparation measures in advance of placing patch 20 on the skin.

In an embodiment of the present invention, transdermal drug delivery product 10 is adapted to be used in conjunction with a system for increasing the permeability of the skin to the drug stored in patch 20, such as by forming microchannels through at least a portion of the skin, and/or by performing iontophoresis and/or by laser ablation and/or by microneedles and/or by sonophoresis and/or by other techniques known in the art. For example, product 10 may be used in conjunction with the ViaDerm drug delivery system, developed by TransPharma Medical Ltd. (Lod, Israel), aspects of which are described in US Patents 6,148,232 and 6,611,706.

In an embodiment of the present invention, moisture of the subject's body comes in contact with the dry drug stored in patch 20, and dissolves the drug to form a saturated solution or suspension. For example, the moisture may be moisture of the skin of the subject. In embodiments of the present invention in which patch 20 is used in conjunction with a device for forming microchannels through the skin, or otherwise ablating or pre-treating the skin, the moisture may be extracellular fluid of the subject released by the body through the microchannels or other openings in the skin.

Although elements of transdermal drug delivery product 10 are shown in the figures as generally circular in shape, this is for illustrative purposes only. For some applications, such elements are elliptical, rectangular, square, or another shape.

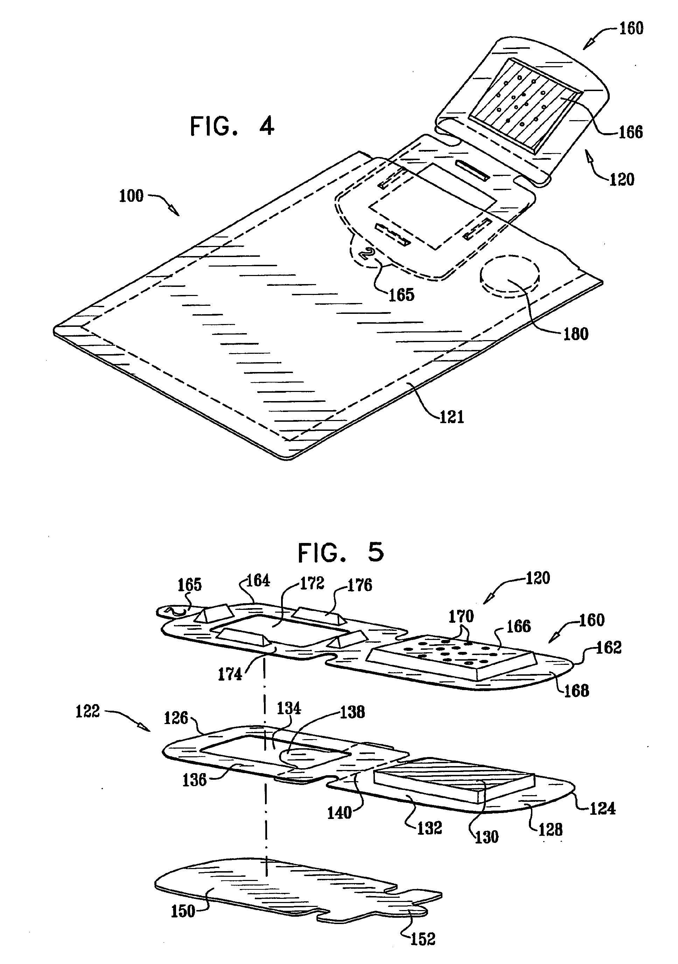

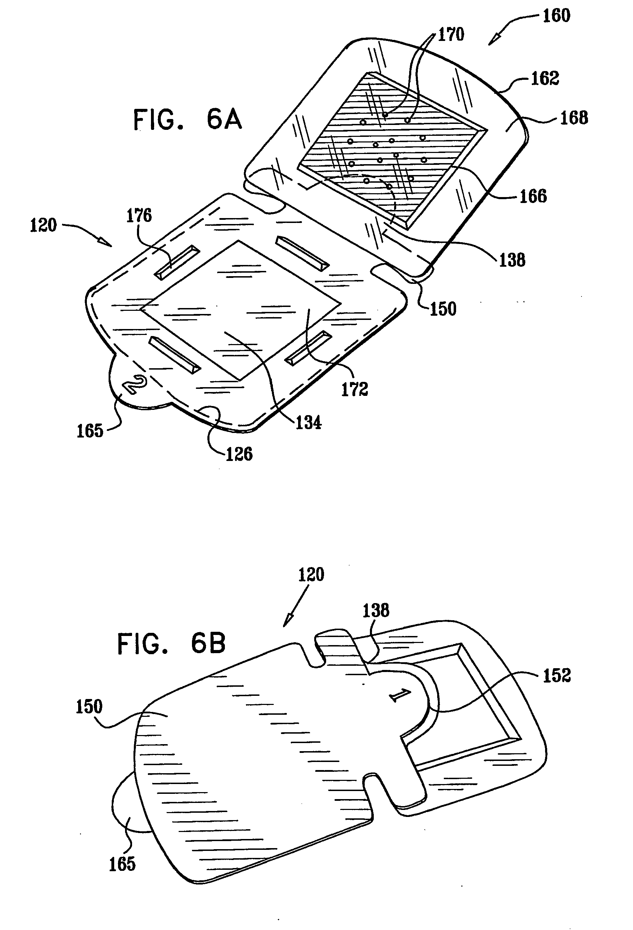

Figs. 4, 5, and 6A-B are schematic illustrations of a transdermal drug delivery product 100, in accordance with an embodiment of the present invention. As shown in Fig. 4, product 100 comprises a patch 120 and a package 121 for storing patch 120 together with its protective covering. Fig. 5 shows the elements of patch 120 prior to assembly thereof, and Figs. 6A and 6B are top and bottom views, respectively, of assembled patch 120.

As can best be seen in Fig. 5, patch 120 comprises an elongated support structure 122, which is shaped so as to define a drug support area 124 and a window area 126. An upper surface 128 of drug support area 124 comprises an adhesive, which serves to: (a) adhere a drug delivery area 130, such as a medicated pad, to drug support area 124, and (b) provide an adhesive border 132, the purpose of which is described below. The scope of the invention includes functionally equivalent alternative configurations of patch 120, such as forming drug delivery area 130 as an integrated component of upper surface 128, e.g., by applying a drug directly to a portion of upper surface 128.

Window area 126 is shaped so as to define a window 134 surrounded by a window frame 136, which comprises an adhesive on both sides thereof. Although window 134 is shown as being square in the figures, the window may also be other shapes, such as rectangular, elliptical, or circular, in which case drug delivery area 130 also typically is a corresponding other shape. Support structure 122 is shaped so as to define a first tab 138, which, prior to assembly, as shown in Fig. 5, may protrude into a portion of window 134. Support structure 122 is configured to have a flexible fold line 140 which separates drug support area 124 from window area 126. For some applications, drug support area 124 and window area 124 are formed from separate pieces, respective portions of which are fixed together to form first tab 138 and join the drug support and window areas together along fold line 140.

Patch 120 further comprises a first liner 150, which is configured to be removably coupled to a lower surface of window frame 136 (which, as mentioned above, comprises an adhesive). During assembly of patch 120, prior to coupling the first liner to the window frame, first tab 138 is folded down and towards drug support area 124 (i.e., to the right in the figure), such that a second tab 152 of first liner 150 partially covers first tab 138, without adhering thereto (such partial covering can best be seen in Fig. 6B). For some applications, first liner 150 comprises a PE/PVC substrate having a thickness of between about 0.07 and about 0.10 mm (about 3 to about 4 mils). Typically, first liner 150 is coated with an easy-release coating, such as an easy-release silicone coating.

Patch 120 still further comprises a second liner 160, which is shaped so as to define a protective area 162, a window area 164, and a third tab 165, which protrudes from window area 164 on the side thereof opposite protective area 162. Protective area 162 is shaped so as to define a raised protective area 166 and a border 168. Raised protective area 166 is configured, upon assembly of the patch, to cover drug delivery area 130 while substantially not coming in contact therewith. Border 168, upon assembly of the patch, is removably coupled to adhesive border 132 of drug support area 124 of support structure 122.

Raised protective area 166 is typically configured to allow gas passage therethrough. Typically, raised protective area 166 is shaped so as to define one or more holes 170 therethrough for this purpose. Package 121 contains a gas, which is typically inert, and may comprise, as appropriate, nitrogen and/or argon. Alternatively, the gas comprises air or another combination of gases.

Window area 164 of second liner 160 is shaped so as to define a window 172 surrounded by a window frame 174, which have approximately the same dimensions as window 134 and window frame 136, respectively, of window area 126 of support structure 122. Upon assembly of patch 120, a lower surface of window frame 174 is removably coupled to the adhesive upper surface of window frame 136, and windows 172 and 134 are generally aligned to define a common window through window areas 164 and 126.

For some applications, second liner 160 comprises a PVC substrate having a thickness of between about 0.1 and about 0.2 mm (about 4 to about 8 mils). Typically, second liner is coated with a moderate-release coating, such as a moderate-release silicone coating. For some applications, window frame 174 is shaped so as to define one or more ridges 176 protruding from the upper surface of the window frame.

Reference is again made to Fig. 4. Package 121 typically comprises a moisture-absorbing desiccant 180, such as a silica gel desiccant. For example, desiccant 180 may comprise a conventional desiccant bag or other desiccant container typically used in drug packaging. For some applications, about 0.25 g to about 3 g of desiccant 180 is provided (e.g., about 0.5 g to about 2 g). Desiccant 180 may be fixed to or removable from package 121, as appropriate. Alternatively or additionally, desiccant 180 is fixed to or integrated with raised protective area 166. For some applications, raised protective area 166 has no holes, and the desiccant is on the face of raised protective area 166 that faces the drug. For other applications, raised protective area 166 has holes, and the desiccant is on the face of raised protective area 166 that is away from the drug, whereby the holes provide fluid communication between the desiccant and the drug. As described hereinabove with respect to protective covering 40 and package 60 of product 10, use of raised protective area 166 allows patch 120 to be packaged immediately upon application of the drug, without waiting for the drug to dry, or within about 30-120 minutes (e.g., 30-60 minutes) following application of the drug, while the drug is only partially dry.

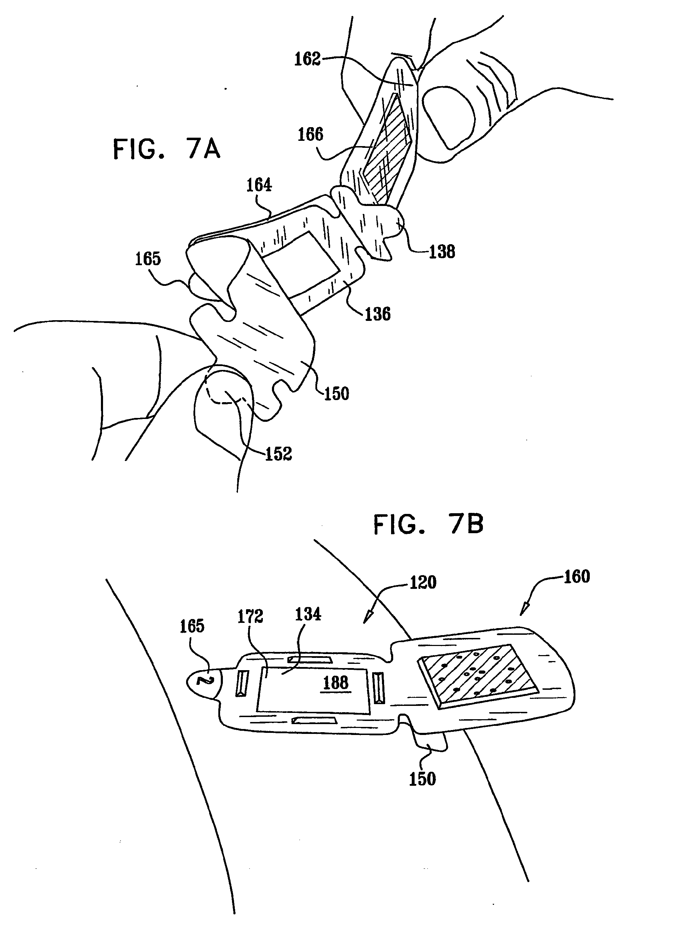

Reference is made to Figs. 7A-E, which are schematic illustrations of application of patch 120 to skin of a subject, in accordance with an embodiment of the present invention. After removing patch 120, together with its protective covering, from package 121 (removal not shown), the subject peels first liner 150 from window frame 136, by grasping second tab 152 of the liner, as shown in Fig. 7A.

As shown in Fig. 7B, the subject applies the patch to skin, such that a portion 188 of the skin is exposed through windows 134 and 172. The adhesive on the lower side of window frame 136 causes the patch to adhere to the skin. For some applications, the skin is cleaned and dried prior to application of the patch. At this point, drug delivery area 130 is facing away from the skin, and is still covered by raised protective area 166. The lower surface of drug support area 124 rests against the skin (with first tab 138 partially intervening), without adhering to the skin.

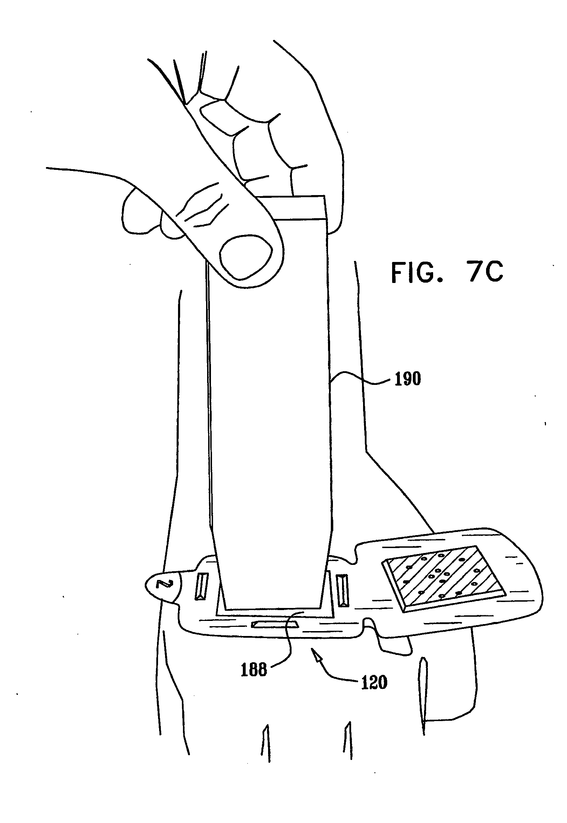

As shown in Fig. 7C, the subject applies an applicator 190 to skin portion 188. Windows 134 and 172 thus serve to guide the user to apply the applicator to an area of the skin precisely located with respect to the other elements of patch 120. Applicator 190 comprises a system for increasing the permeability of the skin to the drug stored in patch 120, such as by forming microchannels through at least a portion of the skin, and/or by performing iontophoresis and/or by laser ablation and/or by microneedles and/or by sonophoresis and/or by other techniques known in the art. For example, applicator 190 may comprise the above-mentioned ViaDerm drug delivery system, aspects of which are described in the above-mentioned US Patents 6,148,232 and 6,611,706.

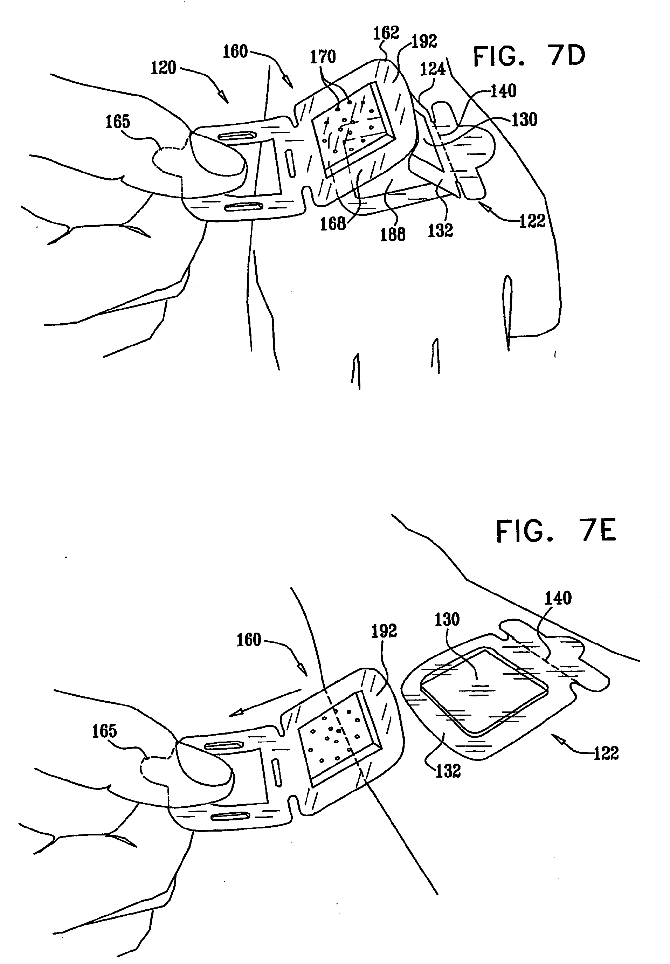

After skin portion 188 has been permeability-enhanced, the subject grasps third tab 165, and pulls second liner 160 towards and over skin portion 188, as shown in Fig. 7D. Such pulling causes three sides of border 168 of protective area 162 to become detached from adhesive border 132 of drug support area 124, while leaving a remaining side 192 of border 168 coupled to the adhesive border.

The subject continues to pull second liner 160, until drug delivery area 130 is completely inverted and is brought in contact with skin portion 188, and side 192 of border 168 becomes detached from adhesive border 132, as shown in Fig. 7E. Support structure 122 is now completed folded along fold line 140, and is adhered to the skin around skin portion 188 by adhesive border 132.

In an embodiment of the present invention, moisture of the subject's body comes in contact with the dry drug stored in patch 120, and dissolves the drug to form a saturated solution or suspension. For example, the moisture may be moisture of the skin of the subject, or extracellular fluid of the subject released by the body through the microchannels or other openings in the skin created by applicator 190.

Although elements of transdermal drug delivery product 100 are shown in the figures as generally rectangular (e.g., square) in shape, this is for illustrative purposes only. For some applications, such elements are elliptical (e.g., circular), or other shapes.

It is noted that, by way of illustration and not limitation, the figures show a relatively small number of holes 50 and 170 that are relatively large. Such configurations typically have between about 3 and 50 or between about 50 and 200 holes that are between about 0.5 and 1 mm or between about 1 and 3 mm in diameter. In other embodiments (not shown), the holes are smaller, e.g., between about 0.01 and 0.1 mm or between about 0.1 and 0.5 mm in diameter. Alternatively or additionally, the number of holes is larger, e.g., between about 200 and 1000, or more than 1000. For example, upper layer 44 (Fig. 1A) or second liner 160 (Figs. 4-6B) may comprise a material such as a cloth, plastic, screen, mesh, porous material, and/or moisture-permeable film, through which a very large number of holes are created or naturally exist. As appropriate, the material may be held taut and/or it may be supported by supporting structures so as to minimize contact between upper layer 44 or raised protective area 166 and the drug.

In an embodiment, techniques and apparatus described herein are combined with techniques and apparatus described in International Application PCT / IL02 / 00896, filed November 7, 2002, which is assigned to the assignee of the present application and is incorporated herein by reference.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. Apparatus comprising a transdermal drug delivery patch product (10, 100), which comprises:
a patch (20, 120), which comprises a drug; and
protective packaging, adapted to store the patch (20, 120), and to allow the drug to dry while the patch is stored in the protective packaging, **characterised in that** the protective packaging comprises:
a removable protective covering (40) applied to the patch (20, 120), configured such that during storage, when the protective covering (40) is applied to the patch, at least a portion of the protective covering (40) is spaced away from the drug, and wherein the protective covering is shaped so as to define one or more holes (50) therethrough, and
a package (60), adapted to store the patch together with the protective covering (40), and comprising a desiccant (62) in fluid communication with the drug via the holes (50) when the patch (20, 120) is stored in the package (60).

2. The apparatus according to claim 1, wherein the protective packaging is configured to dry the drug to a moisture content of 2-3% by weight while the patch (20, 120) is stored in the protective packaging.

3. The apparatus according to claim 1, wherein the protective packaging is configured to dry the drug to a moisture content of 3-5% by weight while the patch (20, 120) is stored in the protective packaging.

4. The apparatus according to claim 1,
wherein the patch (20, 120) is shaped so as define:
a first portion shaped so as to define a frame (136) that surrounds a window (134), and
a second portion, adjacent to the first portion, that defines a drug delivery area (130) comprising the drug, and
wherein the patch (20,120) is configured such that when the second portion is folded onto the first portion, the drug delivery area (130) is placed through the window (134).

5. The apparatus according to claim 1, wherein the protective covering (40) comprises exactly one element.

6. The apparatus according to claim 5, wherein the element is shaped so as to define a peripheral area, and a raised central area shaped so as not to come in contact with the drug.

7. The apparatus according to claim 1, wherein the protective covering (40) comprises a desiccant (62).

8. The apparatus according to claim 7, wherein the desiccant (62) is disposed on a face of the protective covering (40) that faces away from the patch (20,120), such that the desiccant is in fluid communication with the drug via the holes (50) when the patch is stored in the protective packaging.

9. The apparatus according to claim 1, wherein the protective covering (40) is shaped so as to define between 1 and 10 holes therethrough.

10. The apparatus according to claim 1, wherein the protective covering (40) is shaped so as to define between 10 and 100 holes therethrough.

11. The apparatus according to claim 1, wherein the protective covering (40) is shaped so as to define greater than 100 holes therethrough.

12. The apparatus according to claim 1, wherein the protective covering (40) comprises at least one item selected from the list consisting of: a cloth, a plastic, a screen, a mesh, a porous material, and a moisture-permeable film, and wherein the one or more holes (50) are holes in the selected item.

13. A method for manufacturing a transdermal drug delivery patch product (10, 100), the method comprising:
applying a drug to a patch (20, 120); and
while at least a portion of the drug has greater than 6% moisture content by weight, storing the patch in protective packaging adapted to dry the drug while the patch is stored in the protective packaging, **characterised in that** the protective packaging includes:
a removable protective covering (40) shaped so as to define one or more holes (50) therethrough, and wherein storing the patch (20, 120) comprises applying the protective covering (40) to the patch such that at least a portion of the protective covering (40) is spaced away from the drug, and
a package (60) which includes a desiccant (62), and wherein storing the patch (20, 120) comprises storing the patch in the package together with the protective covering such that the desiccant (62) is in fluid communication with the drug via the holes (50).

14. The method according to claim 13, wherein storing the patch (20, 120) in the protective packaging comprises placing the desiccant (62) in the protective packaging.

15. The method according to claim 13 or 14, wherein storing the patch (20, 120) comprises storing the patch (20, 120) in the protective packaging, the protective packaging being adapted to dry the drug to a moisture content of 2-3% by weight while the patch (20, 120) is stored in the protective packaging.

16. The method according to claim 13 or 14, wherein storing the patch (20, 120) comprises storing the patch (20, 120) in the protective packaging, the protective packaging being adapted to dry the drug to a moisture content of 3-5% by weight while the patch (20, 120) is stored in the protective packaging.

17. The method according to claim 13, wherein a desiccant (62) is disposed on a face of the protective covering that faces away from the patch (20,120), and wherein storing the patch comprises applying the protective covering (40) to the patch such that the desiccant is in fluid communication with the drug via the holes (50).

## Patentansprüche

1. Vorrichtung, die ein transdermales Medikamentabgabepflasterprodukt (10, 100) umfasst, das Folgendes umfasst:
ein Pflaster (20, 120), das ein Medikament umfasst, und
eine Schutzverpackung, die dafür ausgelegt ist, das Pflaster (20, 120) zu lagern und zu ermöglichen, dass das Medikament trocknet, während das Pflaster in der Schutzverpackung gelagert wird, **dadurch gekennzeichnet, dass** die Schutzverpackung Folgendes umfasst:
eine abnehmbare Schutzabdeckung (40), die auf dem Pflaster (20, 120) aufgetragen ist und die so konfiguriert ist, dass während der Lagerung, wenn die Schutzabdeckung (40) auf dem Pflaster aufgetragen ist, mindestens ein Teil der Schutzabdeckung (40) von dem Medikament beabstandet angeordnet ist, und wobei die Schutzabdeckung so geformt ist, dass sie ein oder mehrere Löcher (50) dort hindurch definiert, und
eine Verpackung (60), die dafür ausgelegt ist, das Pflaster zusammen mit der Schutzabdeckung zu lagern (40) und die ein Trockenmittel (62) in Fluidverbindung mit dem Medikament über die Löcher (50) umfasst, wenn das Pflaster (20, 120) in der Verpackung (60) gelagert wird.

2. Vorrichtung nach Anspruch 1, wobei die Schutzverpackung so konfiguriert ist, dass sie das Medikament auf einen Feuchtigkeitsgehalt von 2-3 Gew.% trocknet, während das Pflaster (20, 120) in der Schutzverpackung gelagert wird.

3. Vorrichtung nach Anspruch 1, wobei die Schutzverpackung so konfiguriert ist, dass sie das Medikament auf einen Feuchtigkeitsgehalt von 3-5 Gew.% trocknet, während das Pflaster (20, 120) in der Schutzverpackung gelagert wird.

4. Vorrichtung nach Anspruch 1,
wobei das Pflaster (20, 120) so geformt ist, dass es Folgendes definiert:
einen ersten Abschnitt, der so geformt ist, dass er einen Rahmen (136) definiert, der ein Fenster (134) umgibt, und
einen zweiten Abschnitt, der benachbart zu dem ersten Abschnitt angeordnet ist und einen Medikamentabgabebereich (130) definiert, der das Medikament enthält, und
wobei das Pflaster (20, 120) so konfiguriert ist, dass, wenn der zweite Abschnitt auf den ersten Abschnitt gefaltet ist, der Medikamentabgabebereich (130) durch das Fenster (134) angeordnet wird.

5. Vorrichtung nach Anspruch 1, wobei die Schutzabdeckung (40) genau ein Element umfasst.

6. Vorrichtung nach Anspruch 5, wobei das Element so geformt ist, dass es einen Umfangsbereich und einen angehobenen zentralen Bereich definiert, der so geformt ist, dass er nicht mit dem Medikament in Kontakt kommt.

7. Vorrichtung nach Anspruch 1, wobei die Schutzabdeckung (40) ein Trockenmittel (62) umfasst.

8. Vorrichtung nach Anspruch 7, wobei das Trockenmittel (62) auf einer Fläche der Schutzabdeckung (40) angeordnet ist, die von dem Pflaster (20, 120) weg gerichtet ist, so dass das Trockenmittel mit dem Medikament über die Löcher (50) in Fluidverbindung steht, wenn das Pflaster in der Schutzverpackung gelagert wird.

9. Vorrichtung nach Anspruch 1, wobei die Schutzabdeckung (40) so geformt ist, dass zwischen 1 und 10 Löcher dort hindurch definiert sind.

10. Vorrichtung nach Anspruch 1, wobei die Schutzabdeckung (40) so geformt ist, dass zwischen 10 und 100 Löcher dort hindurch definiert sind.

11. Vorrichtung nach Anspruch 1, wobei die Schutzabdeckung (40) so geformt ist, dass mehr als 100 Löcher dort hindurch definiert sind.

12. Vorrichtung nach Anspruch 1, wobei die Schutzabdeckung (40) mindestens ein Element umfasst, das aus der Liste ausgewählt ist, die aus Folgendem besteht: ein Tuch, ein Kunststoff, ein Sieb, ein Netz, ein poröses Material und eine feuchtigkeitsdurchlässige Folie und wobei das eine oder die mehreren Löcher (50) Löcher in dem ausgewählten Element sind.

13. Verfahren zur Herstellung eines transdermalen Medikamentabgabepflasterprodukts (10, 100), wobei das Verfahren Folgendes umfasst:
Auftragen eines Medikaments auf ein Pflaster (20, 120) und
während mindestens ein Teil des Medikaments mehr als 6 % Feuchtigkeitsgewichtsgehalt aufweist, Lagern des Pflasters in einer Schutzverpackung, die dafür ausgelegt ist, das Medikament zu trocknen, während das Pflaster in der Schutzverpackung gelagert wird, **dadurch gekennzeichnet, dass** die Schutzverpackung Folgendes umfasst:
eine abnehmbare Schutzabdeckung (40), die so geformt ist, dass sie ein oder mehrere Löcher (50) dort hindurch definiert, und wobei das Lagern des Pflasters (20, 120) das Auftragen der Schutzabdeckung (40) auf das Pflaster umfasst, so dass mindestens ein Teil der Schutzabdeckung (40) von dem Medikament beabstandet angeordnet ist, und
eine Verpackung (60), die ein Trockenmittel (62) umfasst und wobei das Lagern des Pflasters (20, 120) das Lagern des Pflasters in der Verpackung zusammen mit der Schutzabdeckung umfasst, so dass das Trockenmittel (62) über die Löcher (50) mit dem Medikament in Fluidverbindung steht.

14. Verfahren nach Anspruch 13, wobei das Lagern des Pflasters (20, 120) in der Schutzverpackung umfasst, dass das Trockenmittel (62) in der Schutzverpackung angeordnet wird.

15. Verfahren nach Anspruch 13 oder 14, wobei das Lagern des Pflasters (20, 120) das Lagern des Pflasters (20, 120) in der Schutzverpackung umfasst, wobei die Schutzverpackung dafür ausgelegt ist, das Medikament auf einen Feuchtigkeitsgehalt von 2-3 Gew.% zu trocknen, während das Pflaster (20, 120) in der Schutzverpackung gelagert wird.

16. Verfahren nach Anspruch 13 oder 14, wobei das Lagern des Pflasters (20, 120) das Lagern des Pflasters (20, 120) in der Schutzverpackung umfasst, wobei die Schutzverpackung dafür ausgelegt ist, das Medikament auf einen Feuchtigkeitsgehalt von 3-5 Gew.% zu trocknen, während das Pflaster (20, 120) in der Schutzverpackung gelagert wird.

17. Verfahren nach Anspruch 13, wobei ein Trockenmittel (62) auf einer Fläche der Schutzabdeckung angeordnet ist, die von dem Pflaster (20, 120) weg gerichtet ist, und wobei das Lagern des Pflasters das Auftragen der Schutzabdeckung (40) auf das Pflaster umfasst, so dass das Trockenmittel mit dem Medikament über die Löcher (50) in Fluidverbindung steht.

## Revendications

1. Appareil comprenant un produit de timbre transdermique de délivrance de médicament (10, 100) qui comprend :
un timbre (20, 120) comprenant un médicament ; et
un emballage protecteur, adapté pour stocker le timbre (20, 120) et permettre au médicament de sécher pendant que le timbre est stocké dans l'emballage protecteur, **caractérisé en ce que** l'emballage protecteur comprend :
un revêtement protecteur amovible (40) appliqué sur le timbre (20, 120), configuré de telle sorte que durant le stockage, quand le revêtement protecteur (40) est appliqué sur le timbre, au moins une partie du revêtement protecteur (40) soit écartée du médicament, et dans lequel le revêtement protecteur est conformé de façon à définir une ou plusieurs perforations (50) à travers lui, et
un boîtier (60) adapté pour stocker le timbre avec le revêtement protecteur (40) et comprenant un dessicatif (62) en communication fluidique avec le médicament par l'intermédiaire des perforations (50) quand le timbre (20, 120) est stocké dans le boîtier (60).

2. Appareil selon la revendication 1, dans lequel l'emballage protecteur est configuré pour sécher le médicament jusqu'à une teneur en humidité de 2 à 3% en poids pendant que le timbre (20, 120) est stocké dans l'emballage protecteur.

3. Appareil selon la revendication 1, dans lequel l'emballage protecteur est configuré pour sécher le médicament jusqu'à une teneur en humidité de 3 à 5% en poids tandis que le timbre (20, 120) est stocké dans l'emballage protecteur.

4. Appareil selon la revendication 1,
dans lequel le timbre (20, 120) est conformé de manière à définir :
une première partie conformée de manière à définir un cadre (136) qui entoure une fenêtre (134) ; et
une seconde partie, adjacente à la première partie, qui définit une zone de délivrance de médicament (130) comprenant le médicament, et
dans lequel le timbre (20, 120) est configuré de telle sorte que lorsque la seconde partie est pliée sur la première partie, la zone de délivrance de médicament (130) est placée à travers la fenêtre (134).

5. Appareil selon la revendication 1, dans lequel le revêtement protecteur (40) comprend exactement un seul élément.

6. Appareil selon la revendication 5, dans lequel l'élément est conformé de manière à définir une zone périphérique, et une zone centrale surélevée conformée de manière à ne pas venir en contact avec le médicament.

7. Appareil selon la revendication 1, dans lequel le revêtement protecteur (40) comprend un dessicatif (62).

8. Appareil selon la revendication 7, dans lequel le dessicatif (62) est disposé sur une face du revêtement protecteur (40) qui est détournée du timbre (20, 120) de telle sorte que le dessicatif soit en communication fluidique avec le médicament par l'intermédiaire des perforations (50) quand le timbre est stocké dans l'emballage protecteur.

9. Appareil selon la revendication 1, dans lequel le revêtement protecteur (40) est conformé de manière à définir entre 1 et 10 perforations à travers lui.

10. Appareil selon la revendication 1, dans lequel le revêtement protecteur (40) est conformé de manière à définir entre 10 et 100 perforations à travers lui.

11. Appareil selon la revendication 1, dans lequel le revêtement protecteur (40) est conformé de manière à définir plus de 100 perforations à travers lui.

12. Appareil selon la revendication 1, dans lequel le revêtement protecteur (40) comprend au moins un article sélectionné dans la liste consistant en : un tissu, un plastique, un tamis, un maillage, un matériau poreux, et un film perméable à l'humidité, et dans lequel les une ou plusieurs perforations (50) sont des perforations dans l'article sélectionné.

13. Procédé de fabrication d'un produit de timbre transdermique de délivrance de médicament (10, 100), le procédé comprenant :
l'application d'un médicament sur un timbre (20, 120) ; et
quand au moins une partie du médicament a une teneur en humidité de plus de 6% en poids, le stockage du timbre dans un emballage protecteur adapté pour sécher le médicament pendant que le timbre est stocké dans l'emballage protecteur, **caractérisé en ce que** l'emballage protecteur comprend :
un revêtement protecteur amovible (40) confirmé de manière à définir une ou plusieurs perforations (50) à travers lui, et dans lequel le stockage du timbre (20, 120) comprend l'application du revêtement protecteur (40) sur le timbre de telle sorte qu'au moins une partie du revêtement protecteur (40) soit écartée du médicament, et
un boîtier (60) qui comporte un dessicatif (62), et dans lequel le stockage du timbre (20, 120) comprend le stockage du timbre dans le boîtier avec le revêtement protecteur de telle sorte que le dessicatif (62) soit en communication fluidique avec le médicament par l'intermédiaire des perforations (50).

14. Procédé selon la revendication 13, dans lequel le stockage du timbre (20, 120) dans l'emballage protecteur comprend le placement du dessicatif (62) dans l'emballage protecteur.

15. Procédé selon la revendication 13 ou 14, dans lequel le stockage du timbre (20, 120) comprend le stockage du timbre (20, 120) dans l'emballage protecteur, l'emballage protecteur étant adapté pour sécher le médicament jusqu'à une teneur en humidité de 2 à 3% en poids tandis que le timbre (20, 120) est stocké dans l'emballage protecteur.

16. Procédé selon la revendication 13 ou 14, dans lequel le stockage du timbre (20, 120) comprend le stockage du timbre (20, 120) dans l'emballage protecteur, l'emballage protecteur étant adapté pour sécher le médicament jusqu'à une teneur en humidité de 3 à 5% en poids tandis que le timbre (20, 120) est stocké dans l'emballage protecteur.

17. Procédé selon la revendication 13, dans lequel un dessicatif (62) est disposé sur une face du revêtement protecteur qui est détournée du timbre (20, 120) et dans lequel le stockage du timbre comprend l'application du revêtement protecteur (40) sur le timbre de telle sorte que le dessicatif soit en communication fluidique avec le médicament par l'intermédiaire des perforations (50).
